Europäisches Patentamt

European Patent Office

Office européen des brevets

(19)

(11) **EP 1 078 641 B1**

(12) **EUROPÄISCHE PATENTSCHRIFT**

(45) Veröffentlichungstag und Bekanntmachung des Hinweises auf die Patenterteilung:
**19.07.2006 Patentblatt 2006/29**

(51) Int Cl.:
***A61M 1/16*** (2006.01)

(21) Anmeldenummer: **00118567.7**

(22) Anmeldetag: **26.08.2000**

(54) **Sicherheitsvorrichtung für eine Blutbehandlungsvorrichtung und Verfahren zur Erhöhung der Sicherheit einer Blutbehandlungsvorrichtung**

Safety device for a blood treatment apparatus and method for increasing the safety of a blood treatment apparatus

Dispositif de sécurité pour un appareil de traitement du sang et méthode pour augmenter la sécurité d'un appareil de traitement du sang

(84) Benannte Vertragsstaaten:
**AT BE CH CY DE DK ES FI FR GB GR IE IT LI LU MC NL PT SE**

(30) Priorität: **27.08.1999 DE 19940624**

(43) Veröffentlichungstag der Anmeldung:
**28.02.2001 Patentblatt 2001/09**

(73) Patentinhaber: **Fresenius Medical Care Deutschland GmbH 61352 Bad Homburg v.d.H. (DE)**

(72) Erfinder:
• **Müller, Carsten 97502 Euerbach (DE)**
• **Bardoz, Christoph 97228 Rottendorf (DE)**

(74) Vertreter: **Luderschmidt, Schüler & Partner GbR Patentanwälte, John-F.-Kennedy-Strasse 4 65189 Wiesbaden (DE)**

(56) Entgegenhaltungen:
**DE-A- 4 239 937        US-A- 5 431 811**

**Beschreibung**

Die Erfindung betrifft eine Sicherheitsvorrichtung für eine extrakorporale Blutbehandlungsvorrichtung, insbesondere eine Hämodialyse, Hämofiltrations- oder Himodiafiltrationsvorrichtung und ein Verfahren zur Erhöhung der Sicherheit einer extrakorporalen Blutbehandlungsvorrichtung.

Zur Entfernung von harnpflichtigen Substanzen und zum Flüssigkeitsentzug werden beim chronischen Nierenversagen verschiedene Verfahren zur operativen Blutreinigung bzw. Blutbehandlung eingesetzt. Bei der Hämodialyse (HD) überwiegt der diffusive Stofftransport, während bei der Hämofiltration (HF) ein konvektiver Stofftransport über die Membran stattfindet. Eine Kombination aus beiden Verfahren ist die Hämodiafiltration (HDF).

Der Entzug von überschüssigem Körperwasser durch Ultrafiltration ist ein wesentlicher Bestandteil der Dialysetherapie. Bei der heutigen Standardtherapie wird entweder eine feste Ultrafiltrationsrate UFR oder aber ein fester zeitlicher Verlauf der Ultrafiltrationsrate (UF-Profil) vorgegeben. Die Menge des während der Behandlung zu ultrafiltrierenden Volumens sowie die Ultrafiltrationsrate wirken sich auf die Flüssigkeitskompartiments des Körpers sowie die Kreislaufregulation aus. Daher ist die Ultrafiltration sicherheitskritisch. Bei unkontrolliertem Flüssigkeitsentzug von einigen 1/h kann bereits nach einigen Minuten ein für die Kreislaufregulation kritisches Blutvolumen erreicht sein und ein schwerer Blutdruckabfall ausgelöst werden, der intensivmedizinische Maßnahmen erforderlich macht.

Die Ultrafiltrationsrate UFR eines Dialysators nimmt proportional mit dem bestehenden Transmembrandruck TMP zu, wobei der Transmembrandruck der Drukkunterschied zwischen dem mittleren, blutseitigen und dem mittleren, dialysatseitigen Druck ist. Dabei gibt der Ultrafiltrationskoeffizient an, welcher Wasserentzug (Ultrafiltrationsmenge) pro Stunde und mmHg Transmembrandruck erzielt werden kann (Dimension ml/h x mmHg). Aus Ultrafiltrationskoeffizient UFK und gewünschtem Wasserentzug ergibt sich somit der einzustellende Transmembrandruck TMP.

Zur Erhöhung der Sicherheit des Patienten verfügen die bekannten Blutbehandlungsvorrichtungen über eine Sicherheitsvorrichtung, die den während der Blutbehandlung gemessenen Transmembrandruck dahingehend überwacht, ob er einen oberen und unteren Grenzwert über bzw. unterschreitet, die einen Fensterbereich definieren. Bei Verlassen des Fensterbereiches wird ein Alarm gegeben. Das Erreichen des oberen Grenzwertes kann auf eine Verstopfung des Dialyators hinweisen, das Erreichen des unteren Grenzwertes auf ein Leck des Flüssigkeitssystems. Bei einer Blutbehandlung mit konstanter Ultrafiltrationsrate UFR wird der Fensterbereich vor Beginn der Blutbehandlung manuell anhand der Meßwerte des Transmembrandrucks eingestellt. Ändert sich die Ultrafiltrationsrate jedoch während der Blutbehandlung, muß das Fenster nachgeführt werden. Bei Blutbehandlungsvorrichtungen, die ein stufenartiges Ultrafiltrationsprofil vorgeben, erfolgt eine automatische Nachführung des Fensters anhand der neuen Meßwerte. Dabei dauert die Zentrierung des Fensters nach einer sprunghaften Änderung der Ultrafiltrationsrate im allgemeinen länger als eine Minute.

Wenn die Regelung der Ultrafiltrationsrate kontinuierlich erfolgen soll, ist es erforderlich, das Fenster ständig nachzuführen. Dauert die Zentrierung des Fensters jedoch mehr als eine Minute, kann die Schutzfunktion ganz oder zumindest teilweise außer Kraft gesetzt sein.

Die DE 34 16 057 A1 beschreibt eine Hämodialysevorrichtung, die über eine programmgesteuerte Einrichtung zur Erzeugung einer veränderbaren Zusammensetzung der Dialysierflüssigkeit verfügt. Zur Überwachung wird während der Behandlung überprüft, ob die Leitfähigkeit der Dialysierflüssigkeit in einem vorgegebenen Fenster liegt. Bei einer Veränderung der Zusammensetzung der Dialysierflüssigkeit wird das Leitfähigkeitsüberwachungsfenster verschoben.

Eine Überwachungseinrichtung für Hämodialysegeräte, die eine für den Patienten gefährliche Ultrafiltrationsrate verhindern soll, ist auch aus der DE 42 39 937 C 2 (nächstliegender Stand der Technik) bekannt. Dieses Schutzsystem basiert auf der Überwachung des Transmembran drucks, der sich bei einem Fehler typischerweise ändert.

Der Erfindung liegt die Aufgabe zugrunde, eine Sicherheitsvorrichtung für eine extrakorporale Blutbehandlungsvorrichtung zu schaffen, die eine Überwachung des Transmembrandrucks auch bei einer kontinuierlichen Regelung der Ultrafiltrationsrate mit hoher Zuverlässigkeit erlaubt. Die Lösung dieser Aufgabe erfolgt erfindungsgemäß mit den im Patentanspruch 1 angegebenen Merkmalen.

Darüber hinaus liegt der Erfindung die Aufgabe zugrunde, ein Verfahren zur Erhöhung der Sicherheit einer extrakorporalen Blutbebandlungsvorrichtung anzugeben, mit dem eine Überwachung des Transmembrandrucks auch bei einer kontinuierlichen Regelung der Ultrafiltrationsrate mit hoher Zuverlässigkeit möglich ist. Diese Aufgabe wird erfindungsgemäß mit den Merkmalen des Patentanspruchs 7 gelöst.

Die Sicherheit des Patienten wird dadurch erhöht, daß der obere und/oder untere Grenzwert für den Transmembrandruck unabhängig von dem gemessenen Transmembrandruck festgelegt wird. Der obere und/oder untere Grenzwert wird auf der Grundlage des berechneten Transmembrandrucks bestimmt Damit können die Grenzwerte ohne zeitliche Verzögerung nach einer Änderung der Ultrafiltrationsrate neu festgelegt werden. Es besteht nicht die Gefahr, daß die Schutzfunktion aufgrund einer zeitlichen Verzögerung ganz oder zumindest teilweise außer Kraft gesetzt wird.

Von Vorteil ist, wenn sowohl ein oberer als auch ein unterer Grenzwert für den Transmembrandruck festgelegt werden. Der obere und untere Grenzwert definieren ein Fenster, das während der Behandlung in Abhängigkeit von der Ultrafiltra-tionsrate nachgeführt wird. Prinzipiell kann auch nur der obere oder der untere Grenzwert festgelegt werden.

Der sich bei der vorgegebenen Ultrafiltrationsrate UFR einstellende Transmembrandruck TMP wird aus dem Quotienten der Ultrafiltrationsrate UFR und dem Ultrafiltrationskoeffizienten UFK bestimmt. Vorteilhafterweise wird der Ultrafiltrationskoeffizient UFK durch Division der zu einem bestimmten Zeitpunkt eingestellten Ultrafiltrationsrate UFR durch den zu diesem Zeitpunkt gemessenen Transmembrandruck TMP berechnet. Die Messung des Transmembrandrucks bei einer bestimmten Ultrafiltrationsrate UFR erfolgt zweckmäßigerweise vor bzw. am Beginn der Blutbehandlung. Prinzipiell ist es aber auch möglich, der Berechnung denjenigen Ultrafiltrationskoeffizienten UFK zugrunde zu legen, der vom Hersteller für die Austauscheinheit angegeben wird.

Die das Fenster definierenden Grenzwerte können aus der Summe bzw. der Differenz des berechneten Transmembrandrucks und einem prozentualen Anteil des Transmembrandrucks berechnet werden. Beispielsweise sollte der berechnete Transmembrandruck nicht mehr als 10 % über bzw. unterschritten werden. Während der Behandlung wird das Fenster in Abhängigkeit von der Ultrafiltrationsrate zu größeren oder kleineren Werte hin verschoben, ohne daß die Breite des Fensters verändert wird.

Bei Über- bzw. Unterschreiten des oberen bzw. unteren Grenzwertes wird zweckmäßigerweise ein Alarm gegeben. Darüber hinaus können noch weitere Vorkehrungen getroffen werden, die eine Gefährdung des Patienten verhindern.

Die Sicherheitsvorrichtung für eine extrakorporale Blutbehandlungsvorrichtung weist eine Einrichtung zum Festlegen des oberen und/oder unteren Grenzwertes für den Transmembrandruck TMP und eine Einrichtung zum Vergleichen des gemessenen Transmembrandrucks mit dem oberen und/oder unteren Grenzwert auf. Die Einrichtung zum Festlegen des oberen und/oder unteren Grenzwertes für den Transmembrandruck weist eine Recheneinheit auf, die den sich bei der vorgegebenen Ultrafiltrationsrate einstellenden Transmembrandruck berechnet und auf der Grundlage des berechneten Transmembrandrucks den unteren und/oder oberen Grenzwert bestimmt. Die Berechnung kann mit einem Microprozessor erfolgen, der in den bekannten extrakorporalen Blutbehandlungsvorrichtungen ohnehin vorhanden ist.

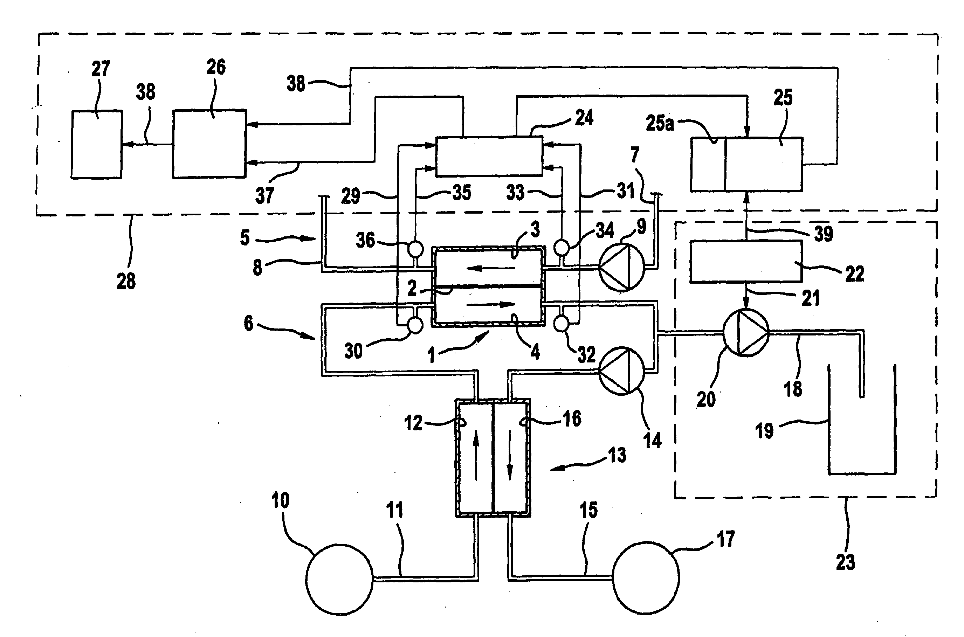

Im folgenden wird ein Ausführungsbeispiel der Erfindung unter Bezugnahme auf die Zeichnung näher erläutert, die die wesentlichen Komponenten einer Hämodialysevorrichtung zusammen mit der Sicherheitsvorrichtung in schematischer Darstellung zeigt.

Die Sicherheitsvorrichtung kann Bestandteil der Hämodialysevorrichtung oder eine separate Einheit sein, die an eine bestehende Hämodiafiltrations-vorrichtung angeschlossen wird.

Die Hämodialysevorrichtung umfaßt einen Dialysator 1, der durch eine semipermeable Membran 2, in eine Blutkammer 3 und eine Dialysierflüssig-keitskammer 4 geteilt ist. Die Blutkammer 3 ist in einen extrakorporalen Blutkreislauf 5 und die Dialysierflüssigkeitskammer 4 in einen Dialysierflüssigkeitskreislauf 6 geschaltet. Von dem Patienten führt eine Blutzuführleitung 7 zu dem Einlaß der Blutkammer 3 des Dialysators 1 und von dem Auslaß der Blutkammer führt eine Blutabfiihrleitung 8 zurück zum Patienten. In die Blutzuführleitung ist eine Blutpumpe 9 geschaltet.

Von einer Dialysierflüssigkeitsquelle 10 führt eine Dialysierflüssigkeitszufiihrleitung 11 zu dem Einlaß der ersten Bilanzkammer 12 einer Bilanziereinheit 13 und von deren Auslaß zu dem Einlaß der Dialysierflüssigkeitskammer 4 des Dialysators 1. Eine Dialysierflüssigkeitsabführleitung 15 führt von dem Auslaß der Dialysierflüssigkeitskammer 4 zu der zweiten Bilanzkammer 16 der Bilanziereinheit 13 und von deren Auslaß zu einem Abfluß 17. In die Dialysierflüssigkeitsabiuhrleitung 15 ist stromauf der Bilanziereinheit 13 eine Dialysierflüssigkeitspumpe 14 geschaltet.

Stromauf der zweiten Bilanzkammer 16 der Bilanziereinheit 13 zweigt von der Dialysierflüssigkeitsabflihrleitung 15 eine Ultrafiltrationsleitung 18 ab, die zu einem Ultrafiltratbehälter 19 führt. Zum Abziehen des Ultrafiltrats ist in die Ultrafiltratleitung 18 eine Ultrafiltratpumpe 20 geschaltet.

Solange die Ultrafiltratpumpe 20 still steht, verhindert die Bilanziereinheit 13 einen Netto-Austausch von Flüssigkeit zwischen Blut- und Dialysierflüssigkeitskreislauf 5, 6. Eine Ultrafiltration findet unter diesen Umständen nicht statt. Die Ultrafiltration wird erst durch das Einschalten der Ultrafiltratpumpe in Gang gesetzt, die aus der Dialysierflüssigkeitskammer 4 des Dialysators 1 gezielt Flüssigkeit entzieht. Die Ultrafiltrationsrate UFR hängt von der Förderrate der Ultrafiltratpumpe 20 ab, die über eine Steuerleitung 21 mit einer Regeleinrichtung 22 verbunden ist. Die Regeleinrichtung 22 verändert die Ultrafiltrationsrate UFR beispielsweise in Abhängigkeit von dem Blutvolumen des Patienten. Die zu der Ultrafiltrationseinrichtung gehörenden Komponenten sind mit den Bezugszeichen 23 versehen.

Die Sicherheitsvorrichtung umfaßt eine Meßeinrichtung 24 zum Messen des Transmembrandrucks TMP, eine Einrichtung 25 zum Festlegen eines oberen und unteren Grenzwertes für den Transmembrandruck TMP, eine Einrichtung 26 zum Vergleichen des gemessenen Transmembrandrucks TMP mit dem oberen und unteren Grenzwert und eine Alarmeinrichtung 27. Die zu der Sicherheitsvorrichtung gehörenden Komponenten sind mit den Bezugszeichen 28 versehen.

Die Meßeinrichtung 24 ist über eine erste Datenleitung 29 mit einem Druckmesser 30 zum Messen des Drucks $P_{Di}$ am Eingang der Dialysierflüssigkeitskammer 4 und über eine Datenleitung 31 mit einem Druckmesser 32 zum Messen des Drucks $P_{Do}$ am Auslaß der Dialysierflüssigkeitskammer 4 des Dialysators verbunden. Über eine Datenleitung 33 ist die Meßeinrichtung 24 mit einem Druckmesser 34 zum Messen des Drucks $P_{Bi}$ am Einlaß der Blutkammer 3 und über eine Datenleitung 35 mit einem Druckmesser 36 zum Messen des Drucks $P_{Bo}$ am Auslaß der Blutkammer 3 des Dialysators 1 verbunden. Aus den gemessenen Werten des Drucks am blut- bzw. dialysatseitigen Ein- bzw. Auslaß des Dialysators 1 berechnet die Meßeinrichtung den Transmembrandruck während der Blutbehandlung wie folgt:

$$TMP = \frac{P_{Bi} + P_{BO}}{2} - \frac{P_{Di} + P_{Do}}{2} \qquad (1)$$

Für den Fall, daß vier getrennte Drucksensoren für die Bestimmung der genannten Größen nicht vorhanden sind, kann der Transmembrandruck TMP auch wie folgt berechnet werden:

$$TMP = P_{BO} - P_{DO} + offset(Q_B, Q_D) \qquad (2)$$

Nach Gleichung (2) kann der Transmembrandruck TMP aus dem Druck $P_{BO}$, $P_{DO}$ am Auslaß der Blut- bzw. Dialysierflüssigkeitskammer 3, 4 des Dialysators 1 und dem Blut- und Dialysierflüssigkeitsfluß $Q_B$, $Q_D$ berechnet werden. Die Funktion offset ist allein von den genannten Flüssen abhängig und berücksichtigt die Druckabfälle längs der Dialysatorkammern.

Die Meßeinrichtung 24 ist mit der Vergleichseinrichtung 26 über eine Datenleitung 37 verbunden. Die Vergleichseinrichtung 26 vergleicht den gemessenen Wert des Transmembrandrucks während der Blutbehandlung mit einem oberen und unteren Grenzwert, die ein Fenster definieren. Wenn der obere Grenzwerte über- bzw. der untere Grenzwert unterschritten werden, erzeugt die Vergleichseinrichtung 26 ein Alarmsignal, das die Alarmeinrichtung 27 über eine Datenleitung 38 empfängt. Die Alarmeinrichtung gibt dann einen akustischen und/oder optischen Alarm, so daß die erforderlichen Vorkehrungen für die Einstellung eines für den Patienten verträglichen Zustandes getroffen werden können.

Der obere und untere Grenzwert für den Transmembrandruck TMP werden während der Behandlung mit der Einrichtung 25 in Abhängigkeit von der Ultrafiltrationsrate festgelegt. Wenn sich die Ultrafiltrationsrate UFR ändert, wird das Fenster nachgeführt. Die Einrichtung 25 empfängt die von der Regeleinrichtung 22 Vorgegebene Ultrafiltrationsrate UFR über die Datenleitung 39. Die Einrichtung 25 verfügt über eine Recheneinheit 25a, in der der obere und untere Grenzwert berechnet werden. Die Berechnung der Grenzwerte wird nachfolgend im einzelnen beschrieben.

Zu Beginn der Behandlung wird in der Recheneinheit 25a der UltrafiltrationskoefEzient UFK aus dem mit der Meßeinrichtung 24 gemessenen Transmembrandruck TMP und der von der Regeleinrichtung 21 vorgegebenen Ultrafiltrationsrate UFR wie folgt berechnet:

$$UFK = \frac{UFR}{TMP} \qquad (3)$$

Während der Behandlung berechnet die Recheneinheit 25a den bei der von der Regeleinrichtung 22 vorgegebenen Ultrafiltrationsrate UFR zu erwartenden Transmembrandruck TMP aus dem zuvor berechneten Ultrafiltrationskoeffizienten UKF und der Ultrafiltrationsrate UFR wie folgt:

$$TMP = \frac{UFR}{UFK} \qquad (4)$$

Der so berechnete Transmembrandruck darf nur um einen bestimmten Prozentsatz über- bzw. unterschritten werden. Den oberen und unteren Grenzwert $L_1$, $L_2$ berechnet die Recheneinheit 25a aus dem gemessenen Transmembrandruck TMP und einem ersten bzw. zweiten Koeffizienten K1, K2 wie folgt:

$$L_1 = TMP + K_1 \cdot TMP \qquad\qquad (5)$$

$$L_2 = TMP - K_2 \cdot TMP \qquad\qquad (6)$$

Den oberen und unteren Grenzwert $L_1$, $L_2$ empfängt die Vergleichseinrichtung 26 über die Datenleitung 38, um den mit der Meßeinrichtung 24 gemessenen Wert des Transmembrandrucks mit dem oberen und unteren Grenzwert vergleichen zu können. Die Berechnung der Fenstergrenzen erfolgt während der Behandlung vorzugsweise in möglichst kurzen Zeitabständen. Zur Bestimmung des zu erwartenden Transmembrandrucks können auch mehrere berechnete Werte herangezogen werden, die nach den bekannten Verfahren einer statistischen Auswertung unterzogen werden.

Die Berechnung des Transmembrandrucks TMP nach Gleichung (4) setzt voraus, daß sich der Transmembrandruck nach Änderung der Ultrafiltrationsrate UFR ohne zeitliche Verzögerung auf den neuen Wert einstellt. In der Praxis stellt sich der neue Transmembrandruck TMP aber mit einer von dem Ultrafiltrationskoeffizienten UFK und der Compliance ("Nachgiebigkeit") des geschlossenen Flüssigkeitssystems (eingeschlossene Gasräume, elastische Schläuche) abhängigen Zeitkonstanten ein. Die Nachführfunktion des Transmembrandrucks ist eine Exponentialfunktion als Lösung einer Differenzialgleichung und nur von der UF-Rate und dem Ultrafiltrationskoeffizienten UFK abhängig. Unter Berücksichtigung der zeitlichen Relaxation aufgrund der Compliance wird der Transmembrandruck über eine Exponentialfunktion und iterativ wie folgt bestimmt:

$$\Delta TMP_i = (1 - e^{-T/\tau})(UFR_{i-1} - UFR_0) / UFK + e^{-T/\tau}\Delta TMP_{i-1} \qquad\qquad (7)$$

Die TMP-Veränderung wird über diskrete Zeitschritte T beispielsweise 5 s aufintegriert. Die Relaxationskonstante $\tau$ wird anhand der bekannten (weil vorher ausgemessenen) Complicance des Systems, die für verschiedene Behandlungsmodi im Gerät hinterlegt sind, und des Ultrafiltrationskoeffizienten UFK berechnet. Der erste Term der Gleichung (7) liefert einen konstanten Beitrag bei der Änderung der UF-Rate und stößt den zweite Term an, der die eigentliche exponentielle Zeitentwicklung enthält. Der zweite Term konvergiert auf einen festen Wert. In der Konvergenz ergibt sich der statische Transmembrandruck.

**Patentansprüche**

**1.** Sicherheitsvorrichtung für eine extrakorporale Blutbehandlungsvorrichtung, die aufweist:

eine durch eine semipermeable Membran in eine erste und eine zweite Kammer getrennte Austauscheinheit, eine Ultrafiltrationseinrichtung zum Entziehen von Ultrafiltrat aus der Austauscheinheit mit einer Ultrafiltrationsrate, die über eine Einrichtung zum Einstellen der Ultrafiltrationsrate verfügt, wobei die Sicherheitsvorrichtung aufweist:

eine Messeinrichtung (24) zum Messen des Transmembrandrucks TMP, eine Einrichtung (25) zum Festlegen eines oberen und/oder unteren Grenzwertes für den Transmembrandruck TMP und eine Einrichtung (26) zum Vergleichen des gemessenen Transmembrandrucks TMP mit dem oberen und/oder unteren Grenzwert, $L_1$, $L_2$

**dadurch gekennzeichnet,**
**dass** die Einrichtung (25) zum Festlegen des oberen und/oder unteren Grenzwertes für den Transmembrandruck TMP eine Recheneinheit (25a) zum Berechnen des sich bei der vorgegebenen Ultrafiltrationsrate UFR einstellenden Transmembrandrucks TMP aufweist, die derart ausgebildet ist, dass der sich bei der vorgegebenen Ultrafiltrationsrate UFR einstellende Transmembrandruck TMP aus dem Quotienten der Ultrafiltrationsrate UFR und dem Ultrafiltrationskoeffizienten UFK bestimmbar ist und der untere und/oder obere Grenzwertes $L_1$, $L_2$ auf der Grundlage

des berechneten Transmembrandrucks bestimmbar ist.

2. Sicherheitsvorrichtung nach Anspruch 1, **dadurch gekennzeichnet, dass** die Recheneinheit (25a) derart ausgebildet ist, dass der Ultrafiltrationskoeffizient UFK durch Bilden des Quotienten der zu einem bestimmten Zeitpunkt eingestellten Ultrafiltrationsrate UFR durch den zu diesem Zeitpunkt gemessenen Transmembrandruck TMP bestimmbar ist.

3. Sicherheitsvorrichtung nach Anspruch 1 oder 2, **dadurch gekennzeichnet, dass** die Recheneinheit (25a) derart ausgebildet ist, dass der obere Grenzwert $L_1$ für den Transmembrandruck TMP aus der Summe des berechneten Transmembrandrucks TMP und dem Produkt des berechneten Transmembrandrucks TMP mit einem ersten Koeffizienten K1 bestimmbar ist.

4. Sicherheitsvorrichtung nach einem der Ansprüche 1 oder 3, **dadurch gekennzeichnet, dass** die Recheneinheit derart ausgebildet ist, dass der untere Grenzwert $L_2$ für den Transmembrandruck TMP aus der Differenz des berechneten Transmembrandrucks TMP und dem Produkt des berechneten Transmembrandrucks TMP mit einem zweiten Koeffizienten K2 bestimmbar ist.

5. Sicherheitsvorrichtung nach einem der Ansprüche 1 bis 4, **dadurch gekennzeichnet, dass** eine Alarmeinrichtung (27) vorgesehen ist, die bei Überschreiten bzw. Unterschreiten des oberen bzw. unteren Grenzwertes einen Alarm gibt.

6. Sicherheitsvorrichtung nach einem der Ansprüche 1 bis 5, **dadurch gekennzeichnet, dass** die Recheneinheit derart ausgebildet ist, dass der Transmembrandruck TMP aus der Ultrafiltrationsrate UFR und dem Ultrafiltrationskoeffizienten UFK über eine Exponentialfunktion iterativ bestimmbar ist.

7. Verfahren zur Erhöhung der Sicherheit einer extrakorporalen Blutbehandlungsvorrichtung, die aufweist:

   eine durch eine semipermeable Membran in eine erste und eine zweite Kammer getrennte Austauscheinheit, eine Ultrafiltrationseinrichtung zum Entziehen von Ultrafiltrat aus der Austauscheinheit mit einer Ultrafiltrationsrate, die über eine Einrichtung zum Einstellen der Ultrafiltrationsrate verfügt, wobei der Transmembrandruck TMP gemessen und ein oberer und/oder unterer Grenzwert für den Transmembrandruck TMP festgelegt wird und der gemessene Transmembrandruck TMP mit dem oberen und/oder unteren Grenzwert verglichen wird,
   **dadurch gekennzeichnet,**
   **dass** der sich bei der vorgegebenen Ultrafiltrationsrate einstellende Transmembrandruck TMP aus dem Quotienten der Ultrafiltrationsrate UFR und dem Ultrafiltrationskoeffizienten UFK berechnet wird und der untere und/oder obere Grenzwert auf der Grundlage des berechneten Transmembrandrucks bestimmt wird.

8. Verfahren nach Anspruch 7, **dadurch gekennzeichnet, dass** der Ultrafiltrationskoeffizient UFK durch Bilden des Quotienten der zu einem bestimmten Zeitpunkt eingestellten Ultrafiltrationsrate UFR durch den zu diesem Zeitpunkt gemessenen Transmembrandruck TMP berechnet wird.

9. Verfahren nach Anspruch 7 oder 8, **dadurch gekennzeichnet, dass** der obere Grenzwert für den Transmembrandruck TMP aus der Summe des berechneten Transmembrandrucks TMP und dem Produkt des berechneten Transmembrandrucks TMP mit einem ersten Koeffizienten K1 berechnet wird.

10. Verfahren nach einem der Ansprüche 7 bis 9, **dadurch gekennzeichnet, dass** der untere Grenzwert für den Transmembrandruck TMP aus der Differenz des berechneten Transmembrandrucks TMP und dem Produkt des berechneten Transmembrandrucks TMP mit einem zweiten Koeffizienten K2 berechnet wird.

11. Verfahren nach einem der Ansprüche 7 bis 10, **dadurch gekennzeichnet, dass** bei Überschreiten bzw. Unterschreiten des oberen bzw. unteren Grenzwertes ein Alarm gegeben wird.

12. Verfahren nach einem der Ansprüche 7 bis 11, **dadurch gekennzeichnet, dass** der Transmembrandruck TMP aus der Ultrafiltrationsrate UFR und dem Ultrafiltrationskoeffizienten UFK über eine Exponentialfunktion iterativ bestimmt wird.

**Claims**

1. A safety device for an extracorporeal blood treatment apparatus, which has:

   an interchangeable unit separated by a semipermeable membrane into a first and a second chamber,
   an ultrafiltration device for the removal of ultrafiltrate from the interchangeable unit with an ultrafiltration rate, which has a device for adjusting the ultrafiltration rate,
   whereby the safety device has:

   a measuring device (24) for measuring transmembrane pressure TMP,
   a device (25) for fixing an upper and/or lower limiting value for transmembrane pressure TMP and
   a device (26) for comparing measured transmembrane pressure TMP with the upper and/or lower limiting value, $L_1$, $L_2$

   **characterised in that**
   the device (25) for fixing the upper and/or lower limiting value for transmembrane pressure TMP has a computing unit (25a) for calculating transmembrane pressure TMP arising with preset ultrafiltration rate UFR, said computing unit being designed in such a way that transmembrane pressure TMP arising with preset ultrafiltration rate UFR can be determined from the quotient of ultrafiltration rate UFR and ultrafiltration coefficient UFK and lower and/or upper limiting value $L_1$, $L_2$ can be determined on the basis of the calculated transmembrane pressure.

2. The safety device according to claim 1, **characterised in that** the computing unit (25a) is designed in such a way that ultrafiltration coefficient UFK can be determined by forming the quotient of ultrafiltration rate UFR arising at a specific time by transmembrane pressure TMP measured at this time.

3. The safety device according to claim 1 or 2, **characterised in that** the computing unit (25a) is designed in such a way that upper limiting value $L_1$ for transmembrane pressure TMP can be determined from the sum of calculated transmembrane pressure TMP and the product of calculated transmembrane pressure TMP with a first coefficient K1.

4. The safety device according to any one of claims 1 or 3, **characterised in that** the computing unit is designed in such a way that lower limiting value $L_2$ for transmembrane pressure TMP can be determined from the difference between calculated transmembrane pressure TMP and the product of calculated transmembrane pressure TMP with a second coefficient K2.

5. The safety device according to any one of claims 1 or 4, **characterised in that** an alarm device (27) is provided, which emits an alarm when the upper or lower limiting value is respectively exceeded or fallen below.

6. The safety device according to any one of claims 1 or 5, **characterised in that** the computing unit is designed in such a way that transmembrane pressure TMP can be determined iteratively from ultrafiltration rate UFR and ultrafiltration coefficient UFK via an exponential function.

7. A method for increasing the safety of an extracorporeal blood treatment apparatus, which has:

   an interchangeable unit separated by a semipermeable membrane into a first and a second chamber,
   an ultrafiltration device for the removal of ultrafiltrate from the interchangeable unit with an ultrafiltration rate, which has a device for adjusting the ultrafiltration rate,
   whereby transmembrane pressure TMP is measured and an upper and/or lower limiting value for transmembrane pressure TMP is fixed and measured transmembrane pressure TMP is compared with the upper and/or lower limiting value,
   **characterised in that**
   transmembrane pressure TMP arising with the preset ultrafiltration rate is calculated from the quotient of ultrafiltration rate UFR and ultrafiltration coefficient UFK and the lower and/or upper limiting value can be determined on the basis of the calculated transmembrane pressure.

8. The method according to claim 7, **characterised in that** ultrafiltration coefficient UFK is calculated by forming the quotient of ultrafiltration rate UFR arising at a specific time by transmembrane pressure TMP measured at this time.

9. The method according to claim 7 or 8, **characterised in that** the upper limiting value for transmembrane pressure TMP is calculated from the sum of calculated transmembrane pressure TMP and the product of calculated transmembrane pressure TMP with a first coefficient K1.

10. The method according to any one of claims 7 to 9, **characterised in that** the lower limiting value for transmembrane pressure TMP is calculated from the difference between calculated transmembrane pressure TMP and the product of calculated transmembrane pressure TMP with a second coefficient K2.

11. The method according to any one of claims 7 to 10, **characterised in that** an alarm is emitted when the upper or lower limiting value is respectively exceeded or fallen below.

12. The method according to any one of claims 7 to 11, **characterised in that** transmembrane pressure TMP is determined iteratively from ultrafiltration rate UFR and ultrafiltration coefficient UFK via an exponential function.

**Revendications**

1. Dispositif de sécurité pour un dispositif de traitement extracorporel du sang qui présente :

   ■ une unité de remplacement séparée par une membrane semi-perméable en une première et une seconde chambres,
   ■ un dispositif d'ultrafiltration pour l'enlèvement de l'ultrafiltrat de l'unité de remplacement avec un taux d'ultrafiltration, qui dispose d'un dispositif pour le réglage du taux d'ultrafiltration,
   ■ le dispositif de sécurité présentant :

     ■ un dispositif de mesure (24) pour la mesure de la pression de la transmembrane TMP,
     ■ un dispositif (25) pour la fixation d'une valeur limite supérieure et/ou d'une valeur limite inférieure pour la pression de la transmembrane TMP et
     ■ un dispositif (26) pour la comparaison de la pression mesurée de la transmembrane TMP avec la valeur limite supérieure et/ou la valeur limite inférieure, $L_1$, $L_2$,

   **caractérisé en ce que** le dispositif (25) pour la fixation de la valeur limite supérieure et/ou de la valeur limite inférieure pour la pression de la transmembrane TMP présente une unité de calcul (25a) pour le calcul de la pression de la transmembrane TMP qui se règle avec le taux d'ultrafiltration UFR prédéfini et qui est conçu de telle sorte que la pression de la transmembrane TMP, qui s'établit avec le taux d'ultrafiltration (UFR) prédéfini, peut être déterminée à partir du quotient du taux d'ultrafiltration UFR et du coefficient d'ultrafiltration UFK et que la valeur limite inférieure et/ou la valeur limite supérieure, $L_1$, $L_2$ peut (peuvent) être déterminée (s) sur la base de la pression calculée de la transmembrane.

2. Dispositif de sécurité selon la revendication 1, **caractérisé en ce que** l'unité de calcul (25a) est conçue de telle sorte que le coefficient d'ultrafiltration UFK peut être déterminé par formation du quotient du taux d'ultrafiltration UFR réglé à un moment défini divisé par la pression de la transmembrane TMP calculée à cet instant.

3. Dispositif de sécurité selon la revendication 1 ou 2, **caractérisé en ce que** l'unité de calcul (25a) est conçue de telle sorte que la valeur limite $L_1$ supérieure pour la pression de la transmembrane TMP peut être déterminée à partir de la somme de la pression calculée de la transmembrane TMP et du produit de la pression calculée de la transmembrane TMP avec un premier coefficient K1.

4. Dispositif de sécurité selon l'une quelconque des revendications 1 ou 3, **caractérisé en ce que** l'unité de calcul est conçue de sorte que la valeur limite inférieure $L_2$ pour la pression de la transmembrane TMP peut être déterminée à partir de la différence de la pression calculée de transmembrane TMP et du produit de la pression calculée de la transmembrane TMP avec un second coefficient K2.

5. Dispositif de sécurité selon l'une quelconque des revendications 1 à 4, **caractérisé en ce qu'**il est prévu un dispositif d'alarme (27) qui émet une alarme lorsque la valeur limite supérieure ou la valeur limite inférieure est atteinte ou n'est pas dépassée.

6. Dispositif de sécurité selon l'une quelconque des revendications 1 à 5, **caractérisé en ce que** l'unité de calcul est conçue de telle sorte que la pression de la transmembrane TMP peut être déterminée de façon itérative à partir du taux d'ultrafiltration UFR et du coefficient d'ultrafiltration UFK au moyen d'une fonction exponentielle.

7. Procédé pour améliorer la sécurité d'un dispositif de traitement extracorporel du sang qui présente :

■ une unité d'échange séparée par une membrane semi-perméable en une première chambre et une seconde chambres,
■ un dispositif d'ultrafiltration pour l'enlèvement de l'ultrafiltrat de l'unité d'échange avec un taux d'ultrafiltration, qui dispose d'un dispositif pour le réglage du taux d'ultrafiltration,
■ la pression de la transmembrane TMP étant mesurée et une valeur limite supérieure et/ou une valeur limite inférieure pour la pression de la transmembrane TMP étant définie et la pression mesurée de la transmembrane TMP étant comparée avec la valeur limite supérieure et/ou la valeur limite inférieure,

**caractérisé en ce que** la pression de la transmembrane TMP, qui s'établit avec le taux d'ultrafiltration prédéfini, est calculée à partir du quotient du taux d'ultrafiltration UFR et du coefficient d'ultrafiltration UFK et la valeur limite inférieure et/ou la valeur limite supérieure est déterminée sur la base de la pression calculée de la transmembrane.

8. Procédé selon la revendication 7, **caractérisé en ce que** le coefficient d'ultrafiltration UFK est calculé par formation du quotient du taux d'ultrafiltration UFR réglé à un instant défini par la pression de la transmembrane TMP mesurée à cet instant.

9. Procédé selon la revendication 7 ou 8, **caractérisé en ce que** la valeur limite supérieure pour la pression de la transmembrane TMP est calculée à partir de la somme de la pression calculée de la transmembrane TMP et du produit de la pression calculée de la transmembrane TMP avec un premier coefficient K1.

10. Procédé selon l'une quelconque des revendications 7 à 9, **caractérisé en ce que** la valeur limite inférieure pour la pression de la transmembrane TMP est calculée à partir de la différence de la pression calculée de la transmembrane TMP et du produit de la pression calculée de la transmembrane TMP avec un second coefficient K2.

11. Procédé selon l'une quelconque des revendications 7 à 10, **caractérisé en ce qu'**une alarme est émise lorsque la valeur limite supérieure ou la valeur limite inférieure est dépassée ou n'est pas atteinte.

12. Procédé selon l'une quelconque des revendications 7 à 11, **caractérisé en ce que** la pression de la transmembrane TMP est calculée de façon itérative à partir du taux d'ultrafiltration UFR et du coefficient d'ultrafiltration UFK au moyen d'une fonction exponentielle.